# EUROPEAN PATENT APPLICATION

(11) **EP 1 864 595 A1**
(43) Date of publication of application: **12.12.2007**
(21) Application number: 07252294.9
(22) Date of filing: 07.06.2007
(51) Int. Cl.: A47D 13/08, A47G 9/10, A61G 7/07, A61F 5/058

(54) **Headrest and method for correcting non-synostatic cranial deformities in infants**

(30) Priority: 08.06.2006 US 449402
(71) Applicant: Tullous, Micam Wade, San Antonio, Texas 78230 (US)
(72) Inventor: Tullous, Micam Wade, San Antonio, Texas 78230 (US)
(74) Representative: Lucking, David John

(57) **Abstract**

The present invention discloses, *inter alia,* a device and method for correcting the shape of an infant's abnormally-shaped cranium by applying external forces over time with the growth of an infant to achieve normal shaping of the infant's head. The device applies inwardly-directed external forces only to areas of bony prominence and minimizes (or altogether eliminates) these forces on the areas of the skull that are less prominent (or flattened). Because the present invention is non-conforming to the shape of an abnormal skull, the exerted forces cause accelerated expansion of the skull in less prominent (flattened) areas coincident with brain and skull growth. This causes the cranium to return to a normal symmetric cranial shape. The material that contacts the infant's cranium is semi-rigid and relatively non-flexible, maintains its overall shape under stress.

## Description

The present invention relates generally to a headrest in which an infant's cranium is positioned while the infant is sleeping to correct cranial deformities. More specifically, the invention relates to a headrest and method for correcting any non-synostotic deformity of the side and posterior aspects of an infant's head.

At birth, the six cranial bones comprising an infant's skull are spaced far enough apart to allow the skull to rapidly grow during the first months of the infant's life. This spacing also allows the bones to overlap so the infant's head can pass through the birth canal without compressing, and thereby damaging, the infant's brain. Eventually-some time between three and six years of age-the cranial bones will fuse and remain fused for the rest of the child's life.

During an infant's normal growth, forces within the infant's skull are directed outward and are constant and equally distributed on the inner surface of the growing skull causing the skull to expand. Accordingly, a decrease of the intracranial pressure will cause a reduced head size. Similarly, an increase in intracranial pressure will cause an increased head size.

Fibrous bands of tissue, called cranial sutures, fill the space between the bones and connect the bones of the skull to each other. These cranial sutures are strong and elastic, providing a flexibility to the skull to allow rapid brain growth during the first months of life. Without the sutures, a child would suffer brain damage due to constriction of the brain during the period of normal growth.

During the first few months of an infants' life, however, the infant is most susceptible to the formation of synostotic or non-synostotic deformities in the cranium. Synostotic deformities are a result of craniosynostosis, which is a birth defect of the skull characterized by premature closure of one or more of the cranial sutures. Craniosynostosis can be hereditary or the result of a metabolic disease, and is characterized by an abnormally-shaped skull and potential for abnormal intracranial pressure, mental retardation, seizures, and blindness.

On the other hand, non-synostotic deformities, in which the cranial sutures remain open, are caused by environmental conditions, including premature birth, torticollis (twisting of the neck muscles beyond their normal position), or the preferred sleeping position of the child. In addition, neurological abnormalities, such as paralysis, cerebral palsy, or some sort of developmental delay, may predispose a child to cranial positioning problems. Non-synostotic deformities are also called positional deformities.

Synostotic and non-synostotic deformities manifest themselves in a variety of ways. Plagiocephaly, for example, is a cranial deformity resulting in an asymmetric head shape. Plagiocephaly consists of a focal area of flattening in the anterior or posterior aspect of one side of the head, which also commonly produces additional compensatory deformities in adjacent areas of the skull, skull base, and face, including the orbital (eye) and mandibular (jaw) structures. This deformity most commonly occurs in the posterior aspect of the head (posterior plagiocephaly), resulting in a focal area of flattening on that side and a compensatory prominence, or bulge, on the other side. In addition, the deformity produces anterior displacement of the ear, ear canal, temporomandibular (jaw) joint, forehead and orbital structures on the same side. Cranial deformities may also be classified, *inter alia,* as brachycephaly (a short, wide head shape), scaphocephaly (a long, narrow head shape), and turricephaly (a pointed head shape).

Non-synostotic posterior plagiocephaly is a very common problem for which parents seek evaluation and recommendations from their family physician or pediatrician. The incidence of this abnormality has increased significantly since publication of recommendations by the American Academy of Pediatrics that neonates (infants) should be put to sleep on their back rather than face down. These recommendations were made to reduce the incidence of Sudden Infant Death Syndrome (SIDS) by eliminating airway and respiratory compromise in the prone (face-down) position, which the Academy considered a possible contributor to the SIDS problem.

The usual method of attempting to treat these deformities involves trying to reposition the child during sleep. The most common adjuncts available to assist with this treatment are flat- and wedge-shaped foam pads. For example, U.S. Patent No. 6,473,923 (filed Nov. 22, 2000) (issued Nov. 5, 2002) discloses a body pillow and head positioner attached to a mat. The device is intended to maintain the infant's supine position while reducing the risk of positional plagiocephaly by causing the head to rotate to the side while maintaining the infant's supine position.

Simply put, repositioning, even with foam padding, is ineffective for treating or preventing these deformities, and even after treatment most children do not obtain a perfectly normal head shape. Repositioning merely distributes or disperses the forces over a larger area of the head. The foam padding remains in contact with the skin and conforms the head to an abnormal shape. Due to this ineffectiveness, a large number of these children require additional treatment from five to ten months of age due to persistent or progressive deformities.

The additional treatment most often is by application of a custom-made external orthosis, or helmet. See, e.g., Corrective Infant Helmet, U.S. Patent No. 6,592,536 (filed Jan. 7, 2000) (issued Jul. 15, 2003); Therapeutic and Protective Infant Helmets, U.S. Patent No. 4,776,324 (filed Apr. 17, 1998) (issued Oct. 11 1998). Such devices provide an expanded area over the site of the deformity, thereby allowing for correction of the deformity over a three to six month period of time related to brain and skull growth and subsequent reshaping. This prolonged time of use is necessary because of the reduced rate of brain and skull growth during the six- to twelve-month time frame. Due to a decrease in the rate of brain and skull growth to approximate fifty percent of the rate from birth to six months and increased stiffness of bones and cranial sutures, the recommendation is to wear the helmet continuously for twenty-three hours each day for up to twelve months. But despite extended use of these helmets, deformities rarely return to a normal shape. In addition, many health insurance companies and programs refuse to pay for these devices, leaving a large number of infants with no available treatment because of the relatively high cost of the helmets.

Another approach to correcting cranial deformities is to soften the material on which the infant's head rests by using a foam pad or memory foam pillow. This method allows the redistribution of inwardly directed forces over a slightly larger surface area, but fails to adequately correct cranial deformities because the softened material conforms to the head shape. The material still contacts, and therefore applies forces to, flattened areas instead of only the abnormal cranial bulges. Preventing cranial deformities with this approach is also ineffective because forces continue to act directly on a focused area of the head. Forces acting on a smaller area of the head results in cranial flattening, and therefore an abnormal head shape, because the head conforms to the shape of the material at the point of contact.

Still another approach is to suspend the infant's head on a flexible material, which, for example, may be a net with an open weave that keeps the infant's head slightly elevated over the resting surface. See Method and Apparatus to Prevent Positional Plagiocephaly in Infants, U.S. Patent No. 6,052,849 (filed Mar. 18, 1999) (issued Apr. 25, 2000). Although the use of an elastic stretchable material or netting may be slightly better than regular foam for preventing the development of flattened areas, these devices do not promote normal shaping due to the continuous application of external forces directed at a smaller posterior aspect of the infant's head. As with the "softened material" approach previously described, forces acting on a smaller area of the head results in cranial flattening because the head conforms to the shape of the stretched material, thus resulting in an abnormal head shape in which the frontal areas are wider than the posterior aspect of the head.

After ten to twelve months of age, little, if any, correction of a cranial deformity can be accomplished with non-operative treatment because of reduced velocity of brain and skull growth, increased thickness of bone, and reduced flexibility of the cranial sutures. Surgical intervention is typically the only effective treatment for moderate to severe deformities in children over twelve months of age.

The prior art for treating this condition does not directly address the cause of the problem, and therefore does not effectively treat the condition. All other products and devices, including foam, elastic (and therefore flexible) material or netting, merely distribute or disperse forces over a larger area of the head. Because these products and devices remain in contact with the skin, they therefore conform the cranium to the abnormal shape. Thus, the prior art does not remove or eliminate the external forces at flattened areas of the cranium, but rather maintains an abnormal cranial shape and promotes a static deformity.

Currently there is no specific apparatus available to provide effective corrective and preventative treatment for non-synostotic cranial deformities in the age range of birth to five months. To avoid the difficulties and pitfalls associated with currently available devices aimed at treating non-synostotic cranial deformities, the present invention discloses a corrective headrest for use at the very first recognition of development of a deformity. The headrest and method allow effective treatment during the rapid period of brain and skull growth (birth to six months), thereby providing rapid correction of the deformity. Children with predisposing conditions possibly require prolonged treatment. Early effective treatment is the key to providing complete correction of these deformities.

According to a first aspect of the invention, we provide a headrest having a semi-rigid body for correcting the shape of an infant's abnormally-shaped cranium comprising:
a bottom surface for contact with a resting surface;
a top surface for contact with said cranium of said infant;
a generally hemi-ellipsoidal depression in said top surface, said depression corresponding to the shape of a normal infantile cranium;
a ridge at one end of said depression for supporting the neck of said infant;
said top surface providing external forces acting on abnormal cranial bulges of said infant's cranium; and
said top surface eliminating external forces acting on abnormal cranial depressions of said infant's cranium.

According to a second aspect of the invention, we provide an apparatus for correcting the shape of an infant's abnormally-shaped cranium comprising:
a first surface;
a generally hemi-ellipsoidal depression in a portion of said first surface;
said depression corresponding to the shape of a normal infantile cranium and having a semi-rigid top surface for supporting said cranium of said infant;
said top surface providing external forces acting on abnormal cranial bulges of said infant's cranium; and
said top surface eliminating external forces acting on abnormal cranial depressions of said infant's cranium.

According to the third aspect of the invention we provide an apparatus for correcting the shape of an infant's abnormally-shaped cranium comprising:
a semi-rigid body;
a hemi-ellipsoidal top surface of said semi-rigid body, said top surface being in the shape of a normal infantile cranium; and
a support means for supporting said semi-rigid body in a position to allow an infant's head to rest on said top surface while said infant is in a generally supine position;
said top surface providing external forces acting on abnormal cranial bulges of said infant's cranium; and
said top surface eliminating external forces acting on abnormal cranial depressions of said infant's cranium.

According to the fourth aspect of the invention, we provide an headrest having a semi-rigid body for preventing abnormal shaping of a normally-shaped infant's cranium comprising:
a bottom surface for contact with a resting surface;
a top surface for contact with said cranium of said infant;
a generally hemi-ellipsoidal depression in said top surface, said depression corresponding to the shape of a normal infantile cranium;
a ridge at one end of said depression for supporting the neck of said infant; and
said top surface contacting and applying pressure to said normally-shaped infant's cranium to prevent development of abnormal cranial bulges and abnormal cranial depressions.

According to a fifth aspect of the invention, we provide an apparatus for preventing development of abnormal shaping of a normally-shaped infant's cranium comprising:
a first surface;
a generally hemi-ellipsoidal depression in a portion of said first surface, said depression corresponding to the shape of a normal infantile cranium and having a semi-rigid top surface for supporting said cranium of said infant; and
said top surface contacting and applying pressure to said normally-shaped infant's cranium to prevent development of abnormal cranial bulges and abnormal cranial depressions.

According to a sixth aspect of the invention, we provide an apparatus for preventing development of abnormal shaping of a normally-shaped infant's cranium comprising:
a semi-rigid body;
a hemi-ellipsoidal top surface of said semi-rigid body, said top surface being in the shape of a normal infantile cranium;
a support means for supporting said semi-rigid body in a position to allow an infant's head to rest on said top surface while said infant is in a generally supine position; and
said top surface contacting and applying pressure to said normally-shaped infant's cranium to prevent development of abnormal cranial bulges and abnormal cranial depressions.

According to a seventh aspect of the invention, we provide a method of correcting an abnormally-shaped cranium of an infant comprising the steps of:
supporting said infant's cranium with a semi-rigid top surface of a headrest, said top surface having a generally hemi-ellipsoidal depression in the shape of a normal infantile cranium;
inhibiting growth of said infant's cranium at abnormal cranial bulges thereof; and promoting growth of said infant's cranium at abnormal cranial depressions thereof.

According to an eighth aspect of the invention, we provide a method of preventing development of abnormal shaping of a normally-shaped infant's cranium comprising the steps of:
placing said infant in a generally supine position;
supporting said normally-shaped infant's cranium in a generally hemi-ellipsoidal depression in a top surface of a headrest; and
applying pressure to said normally-shaped infant's cranium through contact with said top surface to prevent the formation of abnormal cranial bulges and abnormal cranial depressions.

Further features of first to eighth aspects of the invention are set out in claims 7 to 25, claims 27 to 34 and claims 36 to 40 appended hereto.

The present invention discloses, *inter alia,* a device and method for correcting and preventing the shape of an infant's abnormally-shaped cranium by applying external forces over time with the growth of an infant to achieve normal shaping of the infant's head. Unlike the prior art, the present invention both 1) prevents abnormal shaping of an infant's cranium by causing even growth of the infant's normally shaped head and 2) provides forces that act unevenly across an abnormally shaped cranium to correct existing cranial deformities. The embodiments of the present invention include a solid, one-piece headrest structure of uniform consistency, which is molded to approximate the posterior and side aspects of the skull and head, with cervical, or neck, support. The material that contacts the infant's cranium is semi-rigid and relatively non-flexible, maintains its overall shape under stress, and demonstrates minimal superficial focal elasticity only at the site of cutaneous contact.

To correct existing cranial deformities, the present invention applies inwardly-directed external forces only to areas of bony prominence and minimizes (or altogether eliminates) these forces on the areas of the skull that are less prominent (or flattened). The present invention is non-conforming to the shape of an abnormal skull. The forces exerted allow for accelerated expansion of the skull in the less prominent (flattened) areas coincident with brain and skull growth, allowing for return to a normal symmetric cranial shape.

In addition, the headrest prevents development of abnormal cranial shaping by providing a round, normally-shaped contour for the posterior and side aspects of the head, even if the head is turned slightly to one side or the other. Because the contour is normally shaped, the entire surface area of the head that rests in the depression contacts the surface of the headrest. Moreover, because the surface is semi-rigid, the surface will allow for even cranial growth over this area of contact, thereby maintaining the infant's normal head shape.

The preferred embodiment of the present invention is made from an impermeable high-density foam, which provides ease of cleaning as well as flame retardant properties. Other embodiments of the present invention are made from other foam variants, including open cell foam covered with a vinyl or other coating or closed cell foam layered over or applied to more rigid solid or hollow plastic (e.g., PVC or nylon).

Therefore, in accordance with one aspect of the present invention, a headrest having a semi-rigid body for correcting the shape of an infant's abnormally-shaped cranium includes a bottom surface for contact with a resting surface; a top surface for contact with the cranium of the infant; a generally hemi-ellipsoidal depression in the top surface; and a ridge at one end of the depression for supporting the neck of the infant. The shape of the depression corresponds to the shape of a normal infantile cranium. The top surface provides external forces acting on abnormal cranial bulges of the infant's cranium and eliminates external forces that act on abnormal cranial depressions of the infant's cranium.

Other features of the headrest include a rim that defines a substantial portion of the depression, as well as the headrest having a side surface between the bottom surface and the top surface. Furthermore, an additional feature of the headrest includes a curved front surface that cradles the shoulders and further supports the neck of the infant.

Non-limiting examples of the invention, as well as further objects and features thereof, are more clearly and fully set forth in the following description of the preferred embodiment, which should be read with reference to the accompanying drawings, wherein:
Figure 1 is a perspective view of the preferred embodiment of the present invention;
Figure 2 is a frontal view of the preferred embodiment of the present invention;
Figure 3 is a sectional view of the headrest along Line 3-3 of Figure 2;
Figure 4 is a sectional view along Line 4-4 of Figure 2;
Figure 5 is perspective view of an alternative embodiment of the present invention;
Figure 6 is a frontal view of the headrest shown in Figure 5;
Figure 7 is a sectional view along Line 7-7 of Figure 5;
Figure 8 is a perspective view of another alternative embodiment of the present invention;
Figure 9 is a sectional view along Line 9-9 of Figure 8;
Figure 10 is yet another embodiment of the present invention; and
Figure 11 is a sectional view along Line 11-11 of Figure 10.

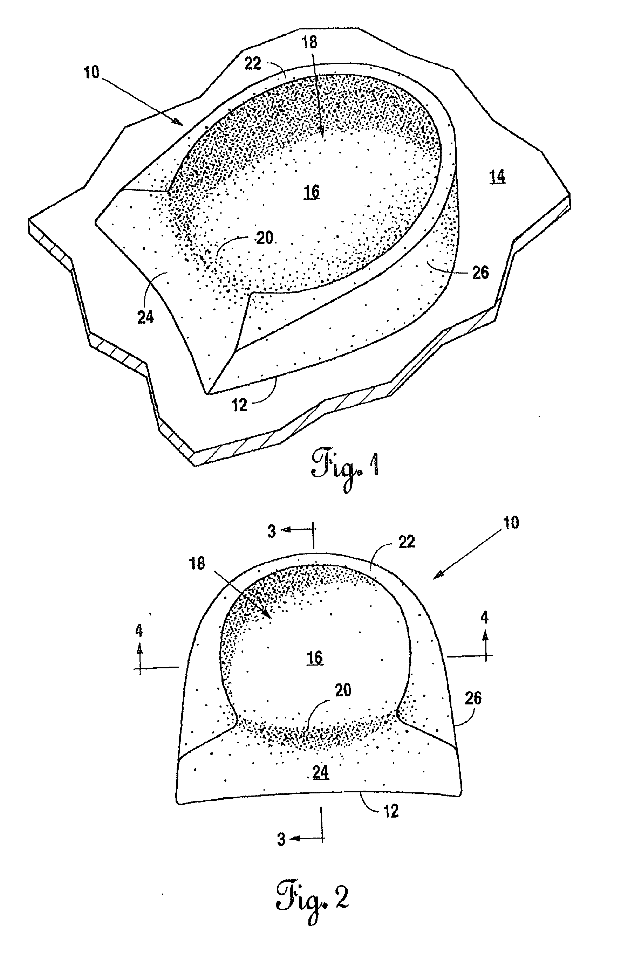

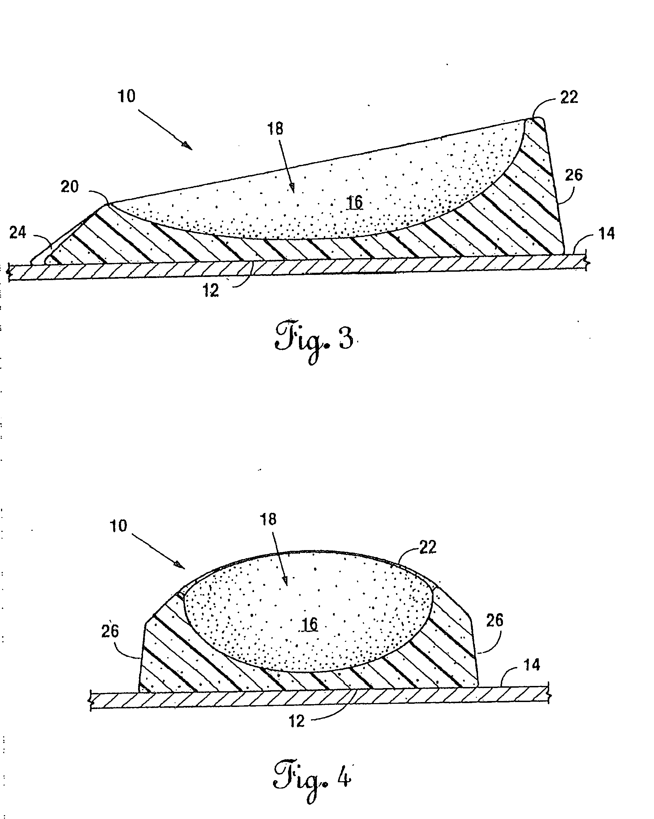

Figure 1 through Figure 4 show a headrest 10 that is the preferred embodiment of the present invention. The headrest 10 comprises a bottom surface 12 for contacting a resting surface 14, and a top surface 16 for contacting an infant's cranium. The top surface 16 comprises a generally hemi-ellipsoidal depression 18, which corresponds to the shape of a normal infantile cranium, and a rim 22 defining a substantial portion of the depression 18. At one end of the depression 18, a ridge 20 is positioned to support the neck of the infant. The top surface 16 is preferably made of a closed cell foam material, but may alternatively be made of open cell foam material covered with a vinyl or other surface coating, closed cell foam layered over higher density foam, open cell foam layered over higher density foam, or closed cell foam layered over a more rigid solid or hollow plastic.

A front surface 24, preferably curved, is positioned to cradle the infant's shoulders and support the neck of the infant while the infant's cranium is in contact with the top surface 16. A preferably-curved side surface 26 extends between the rim 22 and the bottom surface 12. In this preferred embodiment, the headrest 10 is a continuous, uniform, solid body. However, it is anticipated that variations of the uniformity or continuity of the body could occur and be utilized.

In normal operation for correction of an abnormally shaped infant cranium, the headrest 10 is placed on the resting surface 14 so that the bottom surface 12 is in contact therewith. The infant's head is then placed in the depression 18 with the infant's head resting on the top surface 16. Initially, the posterior and part of the side aspects of the infant's head contacts the top surface 16, although during the sleep period the infant's head may roll to one side or the other. Throughout the sleep period, the infant's neck is supported by the ridge 20. The infant's shoulders are aligned in and cradled by the curved front surface 24. As the infant's head makes contact with the top surface 16, the top surface 16 provides external forces acting on any abnormal bulges of the infant's cranium and eliminates external forces that act on abnormal depressions of the infant's cranium. This contact reduces the net outward forces from brain and skull growth at these prominences, and redirects the growth to areas of the cranium where the infant's head is not in contact with the top surface 16.

The headrest 10 works similarly to prevent cranial deformities. The infant's head is placed in the depression 18, which matches the round, normally-shaped contour of the posterior and side aspects of the head. This normally-shaped semi-rigid contour allows for the development of normal cranial shaping regardless of the head's resting position by preventing growth in the areas of contact. Forces from the top surface 16 act on the areas of the cranium in contact therewith, which in an already normally-shaped head is the totality of the surface area resting in the depression 18. Because of its semi-rigid character, the top surface 16 allows the infant's cranium to grow evenly and maintain its normal shape.

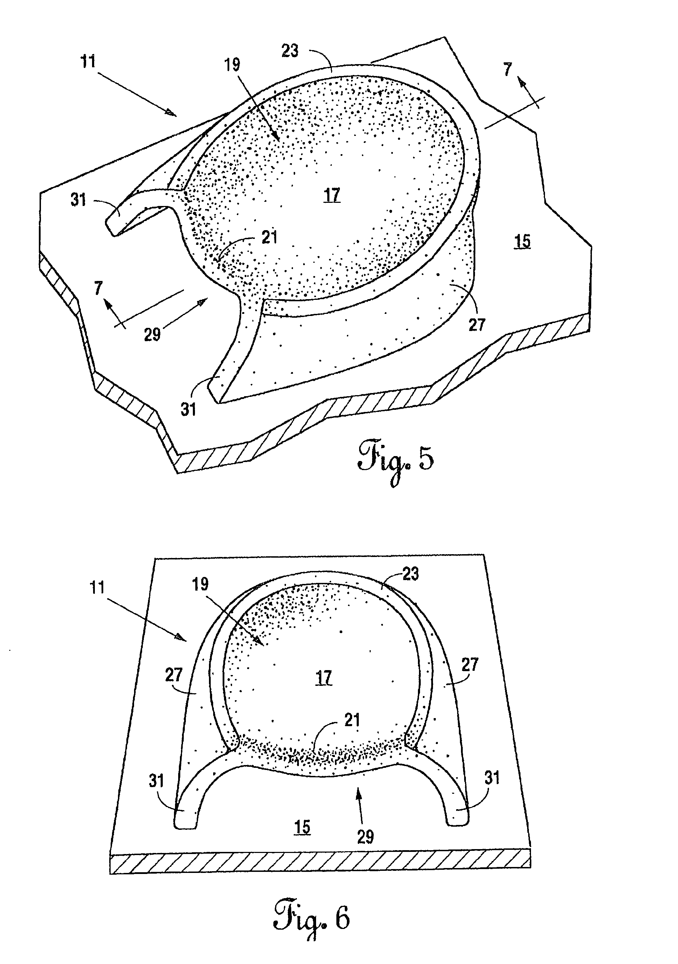

Figure 5 through Figure 7 show an alternative embodiment of the present invention that requires less material to manufacture. The headrest 11 comprises two beams 31 for contacting a resting surface 15, and a top surface 17 for contacting an infant's cranium. The elongated beams 31 are positioned along opposite sides of the headrest 11. The front and back of the headrest 11 are open, forming an opening 29 defined on either side by the beams 31.

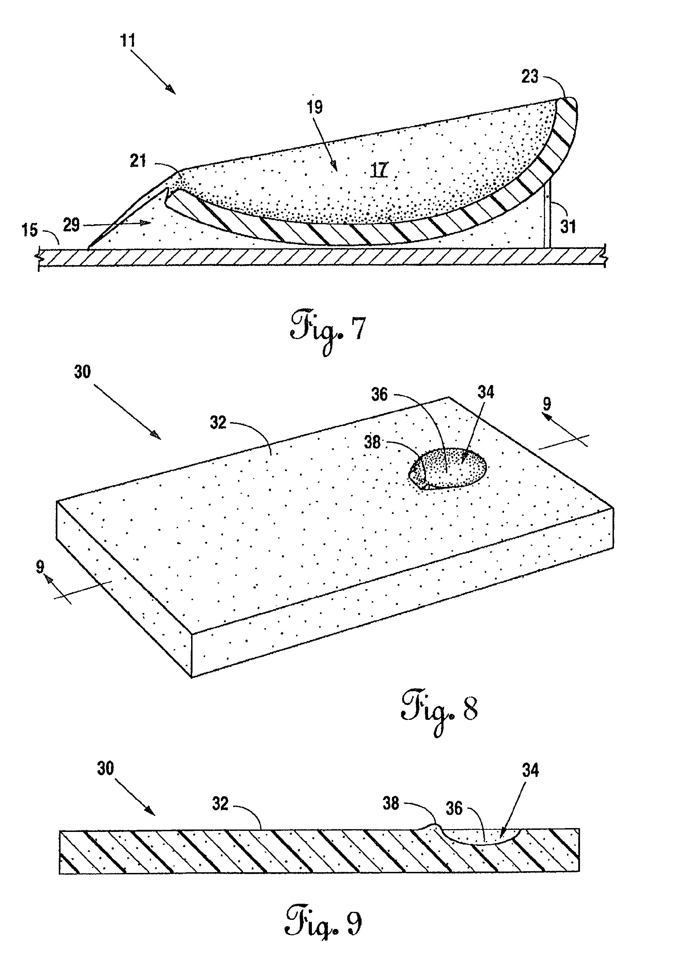

The top surface 17 of the headrest 11 comprises a generally hemi-ellipsoidal depression 19 that corresponds to the shape of a normal infantile cranium and a rim 23 that defines a substantial portion of the depression 19. At one end of the depression 19, a ridge 21 is positioned to support the neck of the infant. The top surface 17 is preferably made of a closed cell foam material, although other materials may be used as described hereinabove. A pair of side surfaces 27, only one of which is shown by Figure 5, adjoin the rim 23 to the beams 31.
As shown more clearly by Figure 7, the beams 31 are positioned at opposing sides of the headrest 11 and along the perimeter thereof, thereby forming an opening 29 between the beams 31. In another embodiment, however, the opposed beams 31 are positioned at the front and rear of the headrest 11.

After placement of the headrest 11 on the resting surface 15 so that the beams 31 are in contact therewith, the infant's head is placed in the depression 19 with the infant's head resting on the top surface 17. Correction and/or prevention of the infant's abnormally shaped cranium is then accomplished in the same manner as in the preferred embodiment.

Figure 8 and Figure 9, which is a sectional view along Line 9-9 of Figure 8, show another embodiment of the present invention. The apparatus of this embodiment comprises a mattress or padded surface 32 and a generally hemi-ellipsoidal depression 34 in a portion of the mattress surface 32. The depression 34 corresponds to the shape of a normal infantile cranium, and has a semi-rigid top surface 36 that is defined by the depression 34. In this embodiment, the top surface 36 is resilient and made of a closed cell foam material, providing external forces acting on abnormal cranial bulges and minimizing external forces acting on abnormal cranial depressions of the infant. However, it is anticipated that other materials could be utilized, such as open cell foam with a vinyl coating. In this embodiment, a ridge 38 at one end of the top surface 36 is shaped and positioned to support the neck of the infant while the infant's head rests on the top surface 36 of the apparatus 30. In another version of this embodiment, it is anticipated that the ridge 38 will be eliminated.

The embodiment shown by Figure 8 and Figure 9 is disclosed with a substantially flat mattress or padded surface 32. However, it is anticipated that the mattress or padded surface 32 could be contoured to prevent an infant from rolling. It is further anticipated that the area of the mattress or padded surface 32 surround the depression 34 could be raised to provide support for the infant's head in a slightly raised position.

As with the already-described embodiments, the infant's head is placed in the depression 34 formed in the mattress 32 such that the infant's head is in contact with the top surface 36. The infant's neck is supported by the ridge 38, while the infant's body is supported in a comfortable resting position by the mattress 32 in a generally supine position. Correction and/or prevention of the infant's abnormally shaped cranium is then accomplished in the same manner as in the preferred embodiment.

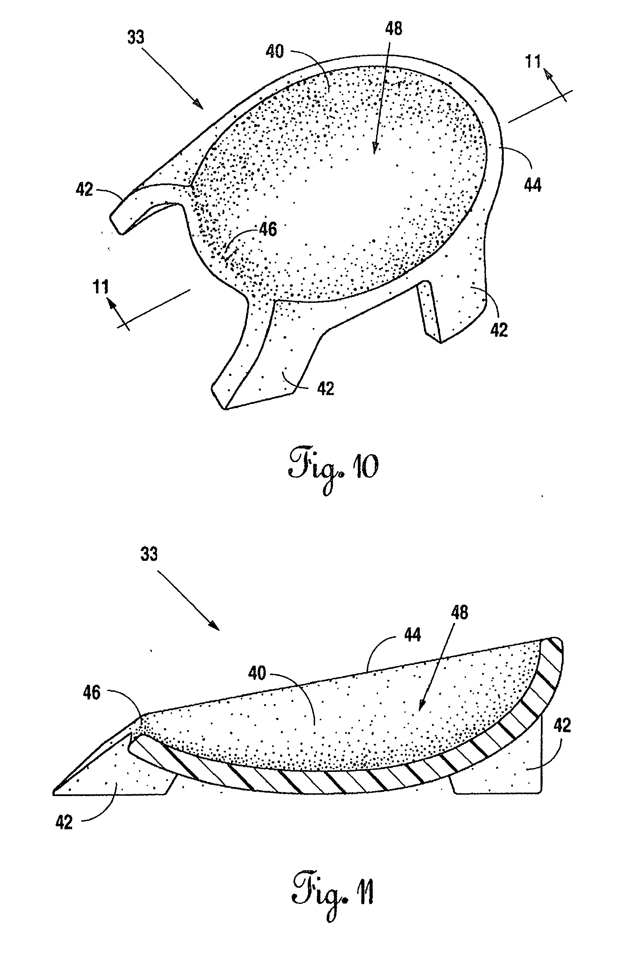

Figure 10 and Figure 11, which is a sectional view along Line 11-11 of Figure 10, show another embodiment of the present invention, an apparatus 33 comprised of a semi-rigid body 44 with a hemi-ellipsoidal top surface 40 that is in the shape of a normal infantile cranium. A plurality of legs 42 support the semi-rigid body 44 in a position to allow an infant's head to rest on the top surface 40. In this embodiment, there are four legs 42, as shown in Figure 10 and Figure 11. However, it is anticipated that more or fewer legs could be used to support the body 44. The top surface 40 is resilient and made of closed cell foam, although in alternative embodiments of the present invention the top surface 40 may be made of other material, including open cell foam covered with a vinyl coating and other materials as described hereinabove. Furthermore, a ridge 46 at one end of the top surface 40 is shaped and positioned to support the neck of the infant while the infant's head rests on the top surface 40 of the apparatus 33.

After placement of the apparatus 33 on a resting surface so that legs 42 are in contact therewith, the infant's head is placed in the depression 48 with the infant's head resting on the top surface 40 and the infant's neck being supported by the ridge 46. Correction and/or prevention of the infant's abnormally shaped cranium is then accomplished in the same manner as in the preferred embodiment.

The present invention is described above in terms of a preferred illustrative embodiment of a specifically described headrest, as well as alternative embodiments of the present invention. Those skilled in the art will recognize that alternative constructions of such a headrest can be used in carrying out the present invention.

Other aspects, features, and advantages of the present invention may be obtained from a study of this disclosure and the drawings, along with the appended claims.

## Claims

1. A headrest having a semi-rigid body for correcting the shape of an infant's abnormally-shaped cranium comprising:
a bottom surface for contact with a resting surface;
a top surface for contact with said cranium of said infant;
a generally hemi-ellipsoidal depression in said top surface, said depression corresponding to the shape of a normal infantile cranium;
a ridge at one end of said depression for supporting the neck of said infant;
said top surface providing external forces acting on abnormal cranial bulges of said infant's cranium; and
said top surface eliminating external forces acting on abnormal cranial depressions of said infant's cranium.

2. An apparatus for correcting the shape of an infant's abnormally-shaped cranium comprising:
a first surface;
a generally hemi-ellipsoidal depression in a portion of said first surface;
said depression corresponding to the shape of a normal infantile cranium and having a semi-rigid top surface for supporting said cranium of said infant;
said top surface providing external forces acting on abnormal cranial bulges of said infant's cranium; and
said top surface eliminating external forces acting on abnormal cranial depressions of said infant's cranium.

3. An apparatus for correcting the shape of an infant's abnormally-shaped cranium comprising:
a semi-rigid body;
a hemi-ellipsoidal top surface of said semi-rigid body, said top surface being in the shape of a normal infantile cranium; and
a support means for supporting said semi-rigid body in a position to allow an infant's head to rest on said top surface while said infant is in a generally supine position;
said top surface providing external forces acting on abnormal cranial bulges of said infant's cranium; and
said top surface eliminating external forces acting on abnormal cranial depressions of said infant's cranium.

4. A headrest having a semi-rigid body for preventing abnormal shaping of a normally-shaped infant's cranium comprising:
a bottom surface for contact with a resting surface;
a top surface for contact with said cranium of said infant;
a generally hemi-ellipsoidal depression in said top surface, said depression corresponding to the shape of a normal infantile cranium;
a ridge at one end of said depression for supporting the neck of said infant; and
said top surface contacting and applying pressure to said normally-shaped infant's cranium to prevent development of abnormal cranial bulges and abnormal cranial depressions.

5. An apparatus for preventing development of abnormal shaping of a normally-shaped infant's cranium comprising:
a first surface;
a generally hemi-ellipsoidal depression in a portion of said first surface, said depression corresponding to the shape of a normal infantile cranium and having a semi-rigid top surface for supporting said cranium of said infant; and
said top surface contacting and applying pressure to said normally-shaped infant's cranium to prevent development of abnormal cranial bulges and abnormal cranial depressions.

6. An apparatus for preventing development of abnormal shaping of a normally-shaped infant's cranium comprising:
a semi-rigid body;
a hemi-ellipsoidal top surface of said semi-rigid body, said top surface being in the shape of a normal infantile cranium;
a support means for supporting said semi-rigid body in a position to allow an infant's head to rest on said top surface while said infant is in a generally supine position; and
said top surface contacting and applying pressure to said normally-shaped infant's cranium to prevent development of abnormal cranial bulges and abnormal cranial depressions.

7. The headrest or apparatus of any one of Claims 1 to 6 wherein said top surface is resilient.

8. The headrest of Claim 1 wherein said semi-rigid body is made of a closed cell foam material.

9. The headrest of Claim 1 or Claim 7 as dependent on Claim 1 wherein said semi-rigid body is made of an open cell foam material and covered with a vinyl coating.

10. The apparatus or headset of any one of Claims 2 to 6 wherein said top surface is a closed cell foam.

11. The apparatus or apparatus of any one of Claims 2 to 6 wherein said top surface is an open cell foam and covered with a vinyl coating.

12. The headrest of any one of Claims 1, 8 or 9 wherein said top surface further comprises a rim defining a substantial portion of said depression.

13. The headrest of Claim 12 further comprising at least one side surface between said bottom surface and said top surface.

14. The headrest of any one of Claims 1, 4, 8, 9, 12 or 13 further comprising a front surface.

15. The headrest of Claim 14 wherein said front surface supports the neck of said infant.

16. The headrest of Claim 15 wherein said front surface is curved to cradle said infant's shoulders.

17. The headrest of any one of Claims 1, 4, 8, 9 or 12 to 16 wherein said resting surface is a mattress surface, a table surface, or a car seat surface.

18. The apparatus of any one of Claims 2, 5, or any one of Claims 7, 10 or 11 as dependent on Claim 2 or Claim 5, further comprising a ridge at one end of said depression, said ridge being shaped and positioned to support the neck of said infant at the base of said infant's cranium.

19. The apparatus of any one of Claims 2, 5, or 18, or any one of Claims 7, 10 or 11 as dependent on Claim 2 or Claim 5, wherein said first surface is a mattress surface, a pad surface, a table surface, or a headrest surface.

20. The apparatus of Claim 3 or Claim 6 further comprising a ridge at one end of said top surface, said ridge being shaped and positioned to support the neck of said infant.

21. The apparatus of Claim 3, Claim 6 or Claim 20 wherein said support means are beams on opposing sides of said semi-rigid body.

22. The apparatus of Claims 3, Claim 6 or Claim 20 wherein said support means is a plurality of legs.

23. The headrest or apparatus of any one of Claims 4, 5, 6, or any one of Claims 7, 10, 11 or 14 to 17 as dependent on Claims 4, 5 or 6, wherein said pressure is isometric.

24. The headrest or apparatus of Claim 4, or any one of Claims 7, 10, 11 or 14 to 17 as dependent on Claim 4, further comprising a rim defining a substantial portion of said depression.

25. The headrest or apparatus of Claim 4, or any one of Claims 7, 10, 11 or 14 to 17 as dependent on Claim 4, further comprising at least one side surface between said bottom surface and said top surface.

26. A method of correcting an abnormally-shaped cranium of an infant comprising the steps of:
supporting said infant's cranium with a semi-rigid top surface of a headrest, said top surface having a generally hemi-ellipsoidal depression in the shape of a normal infantile cranium;
inhibiting growth of said infant's cranium at abnormal cranial bulges thereof; and
promoting growth of said infant's cranium at abnormal cranial depressions thereof.

27. The method of Claim 26 further comprising the step of supporting the neck of said infant with a ridge at one end of said depression.

28. The method of Claim 26 or Claim 27 further comprising the step of further supporting the neck of said infant with a front surface of a said semi-rigid body.

29. The method of any one of Claims 26 to 28 further comprising the step of positioning the shoulders of said infant in a curved portion of said front surface.

30. The method of any one of Claims 26 to 29 further comprising the step of placing a headrest on a resting surface wherein a bottom surface of said headrest contacts said resting surface.

31. The method of any one of Claims 26 to 30 wherein the step of inhibiting growth at said cranial bulges further comprises the step of applying forces to act on said cranial bulges of said infant's cranium through contact with said top surface.

32. The method of Claim 31 wherein said forces are sufficient to resist the outwardly-directed intracranial pressure acting on said infant's cranium and direct said intracranial pressure to the location of said cranial depressions.

33. The method of any one of Claims 26 to 32 wherein the step of promoting growth at cranial depressions further comprises the step of minimizing support forces that act on said cranial depressions of said infant's head through said top surface.

34. The method of Claim 33 wherein said forces acting on said cranial depressions are eliminated.

35. A method of preventing development of abnormal shaping of a normally-shaped infant's cranium comprising the steps of:
placing said infant in a generally supine position;
supporting said normally-shaped infant's cranium in a generally hemi-ellipsoidal depression in a top surface of a headrest; and
applying pressure to said normally-shaped infant's cranium through contact with said top surface to prevent the formation of abnormal cranial bulges and abnormal cranial depressions.

36. The method of Claim 35 wherein said pressure is isometric.

37. The method of Claim 35 or Claim 36 further comprising the step of supporting the neck of said infant with a ridge, said ridge being located at one end of said depression.

38. The method of Claim 37 further comprising the step of supporting the neck of said infant with a front surface of said headrest.

39. The method of Claim 37 or Claim 38 further comprising the step of positioning the shoulders of said infant in a curved portion of said front surface.

40. The method of any one of Claims 37, 38 or 39 further comprising the step of placing a headrest on a resting surface wherein a bottom surface of said headrest contacts said resting surface.
